# EUROPEAN PATENT APPLICATION

(11) **EP 4 537 859 A1**
(43) Date of publication of application: **16.04.2025**
(21) Application number: 23819310.6
(22) Date of filing: 06.06.2023
(51) Int. Cl.: A61L 24/06, A61K 35/16, A61K 35/19

(54) **FIBRIN GEL AND METHOD FOR OBTAINING SAME**

(30) Priority: 07.06.2022 ES 202230498
(71) Applicant: Santxa Research S.L.U., 01008 Vitoria-Gasteiz (ES)
(72) Inventor: SÁNCHEZ ÁLVAREZ, Mikel, 01008 Vitoria-Gasteiz, Álava (ES); DELGADO SAN VICENTE, Diego, 01008 Vitoria-Gasteiz, Álava (ES); BEITIA SAN VICENTE, Maider, 01008 Vitoria-Gasteiz, Álava (ES); SÁNCHEZ ARIZMENDIARRIETA, Pello, 01008 Vitoria-Gasteiz, Álava (ES)
(74) Representative: ABG Intellectual Property Law, S.L.
(86) International application number: PCT/ES2023/070376
(87) International publication number: WO 2023/237799

(57) **Abstract**

The present invention relates to a method for producing a fibrin gel, to the gel obtained using the method and to the use thereof in osteochondral injuries, meniscal injuries or ulcers. The invention also relates to a combination of a fibrinogenic concentrate and a platelet-free serum comprising thrombin and growth factors, for use in medicine.

## Description

### FIELD OF THE INVENTION

The present invention belongs to the field of biotechnology and relates to a method for obtaining a fibrin and growth factors gel, and to the gel obtained using the method.

### BACKGROUND OF THE INVENTION

Fibrin gels are a type of surgical sealant or "glue". The ingredients of these sealants interact during application to form a stable clot made up of a blood protein called fibrin.

Fibrin gels are currently used during surgery for several different purposes: to control bleeding in the area where the surgeon is operating, to accelerate wound healing, to seal hollow body organs or cover holes made with standard sutures, to provide slow release of medicinal products to exposed tissues during surgery.

Fibrin gels generally consist of two components derived from human plasma: (a) a highly concentrated fibrinogen complex (FC) primarily made up of fibrinogen and fibronectin along with catalytic amounts of factor XIII and plasminogen and (b) a high potency thrombin.

By the action of thrombin, (soluble) fibrinogen is first converted into fibrin monomers that spontaneously aggregate and form the so-called fibrin clot. Simultaneously, factor XIII (FXIII) present in solution is activated by thrombin in the presence of calcium ions to factor Xllla. The aggregated fibrin monomers and any remaining fibronectin possibly present are cross-linked to form a high polymer by means of the formation of new peptide bonds. By means of this cross-linking reaction, the strength of the clot formed substantially increases. Generally, the clot adheres well to wound and tissue surfaces, leading to the adhesive and hemostatic effect (U.S. Patent 7,241,603). Therefore, fibrin gels are frequently used as two-component adhesives comprising a fibrinogen complex (FC) component together with a thrombin component further containing calcium ions.

A particular advantage of a fibrin gel is that the adhesive/gel does not remain at its application site as a foreign body, but is completely resorbed as in natural wound healing and replaced by newly formed tissue. Various cells, for example macrophages and subsequently fibroblasts, migrate into the gel, lyse and resorb material from the gel and form new tissue. Fibrin sealants have been used to form fibrin gels *in situ,* and these fibrin gels have been used to deliver cells and growth factors (Cox et al., Tissue Eng 10:942-954, 2004; Wong et al., Thromb Haemost 89 :573-582, 2003).

However, when these gels are used as sealants or therapeutic agents and *in situ* polymerization of these gels is performed by means of mixing their main components, fibrinogen and thrombin, the resulting gel does not retain all the properties of naturally formed clots, since, in most cases they are not completely of natural origin and this may trigger rejection or biosafety processes due to possible contaminations. Furthermore, when, in addition to the hemostatic effect, the aim is to favor regeneration or healing processes by means of incorporating growth factors into said gels, these are restricted to isolated factors, but there are no gels available that retain the wide range of growth factors and cytokines that are present in clots formed endogenously by the organism itself.

Therefore, there is still a need in the art to develop improved methodologies for the formation of fibrin gels that retain all the properties in terms of composition and regenerative activity of natural clots and that can be used in a controlled and localized manner in the treatment of injuries or pathologies that require the regenerative and healing properties of said clots.

### SUMMARY OF THE INVENTION

The development of a new methodology for the production of a completely natural fibrin gel which is obtained from a patient's blood is shown herein. This allows a high level of biosafety and utilizes the biological properties of the biomolecules present in plasma.

As shown in the examples of the application, the authors of the present invention have demonstrated that the fibrin gel which is obtained by means of the proposed methodology results in a gel that does not sustain retraction over time and has a higher adhesion strength in comparison with a conventional fibrin clot. In this regard, the authors of the invention propose a methodology in which the gel would be obtained by means of the mixture of a fibrinogenic concentrate and a solution of thrombin and growth factors both of which are isolated from a subject's blood. Furthermore, the inventors have demonstrated that the removal of an excessive concentration of platelets from the fibrinogenic concentrate prevents the formation of clots in said fibrinogenic concentrate, which clots hinder the formation of the end product and give rise to the generation of a heterogeneous gel. Moreover, the formation of clots due to the presence of platelets seems to affect the adhesion capacity as suggested by the data relating to adhesion strength, which is significantly higher in the gel without platelets.

Polymerization of the gel occurs within the first two minutes of mixing the components, resulting in an easily applicable gel that adheres to the tissue and remains consistent.

In that sense, in a first aspect the invention relates to an *"in vitro"* method for producing a fibrin gel comprising the following steps:
a) obtaining a platelet-poor plasma (PPP) from an anticoagulated blood sample from a subject,
b) processing the PPP to obtain a first composition comprising a fibrinogenic concentrate,
c) obtaining a platelet-rich plasma (PRP) from an anticoagulated blood sample from a subject,
d) activating the platelets in the PRP obtained in c),
e) isolating the serum obtained after platelet activation to obtain a second platelet-free composition comprising thrombin and growth factors,
f) mixing the first composition and the second composition, preferably at a temperature of between 30°C and 44°C.

In a second aspect, the invention relates to a fibrin gel obtained by means of the method of the first aspect of the invention.

In a third aspect, the invention relates to the fibrin gel of the second aspect of the invention for use in medicine.

In another aspect, the invention relates to the fibrin gel of the second aspect of the invention for use in the treatment of osteochondral injuries, meniscal injuries or ulcers, particularly vascular ulcers.

### BRIEF DESCRIPTION OF THE FIGURES

**Figure 1****.** "Non-retraction" capacity of the new formulation in comparison with conventional formulations at the macroscopic level (A) and microscopic level (B).
**Figure 2****.** Adherence capacity of the new formulation in real tissue (cartilage).
**Figure 3****.** Cell viability assay with calcein/ethidium to label live cells (left) and dead cells (right).
**Figure 4****.** Percentage of coagulation of the new formulation according to the temperature.
**Figure 5****.** Influence of the removal of platelets in the new formulation at the macroscopic level and homogeneity of the fibrinogenic concentrate and the final clot.
**Figure 6****.** Influence of the removal of platelets in the new formulation on the adherence strength.

### DETAILED DESCRIPTION OF THE INVENTION

All terms as used in the present application, unless otherwise indicated, shall be understood in their common meaning as known in the art. Other more specific definitions for certain terms as used in the present application will be defined later in this section and are intended to be applied uniformly throughout the specification and claims, unless a definition expressly set forth otherwise provides a broader definition.

### Method of the invention

In a first aspect, the invention relates to an *"in vitro"* method for producing a fibrin gel comprising the following steps:
a) obtaining a platelet-poor plasma (PPP) from an anticoagulated blood sample from a subject,
b) processing the PPP to obtain a first composition comprising a fibrinogenic concentrate,
c) obtaining a platelet-rich plasma (PRP) from an anticoagulated blood sample from a subject,
d) activating the platelets in the PRP obtained in c),
e) isolating the serum obtained after platelet activation to obtain a second platelet-free composition comprising thrombin and growth factors,
f) mixing the first composition and the second composition.

In a preferred embodiment, the method, in step f), comprises mixing the first composition and the second composition at a temperature of between 30°C and 44°C, for example at about 37°C.

The term *"in vitro",* as used herein, refers to the method not being carried out in the body of a human or animal subject, but rather in cells or fluids isolated from said subject or in a test tube.

The term "fibrin", as used herein, refers to a fibrillar protein with the ability to form three-dimensional networks. This protein plays an important role in the coagulation process in view of its properties. It has the structure of a pole with three globular areas. It has the ability to create aggregates with other molecules that form a soft clot. It is not normally found in the blood, it is formed from circulating fibrinogen, which, by means of the action of the enzyme called thrombin, is converted into fibrin, which has coagulation effects.

"Fibrin gel", also known as "fibrin matrix", "fibrin glue", "fibrin tissue adhesive", "fibrin sealant" or "fibrin scaffold" refers to a hemostatic agent in the form of a semi-solid gel (semi-solid composition) derived from plasma coagulation proteins.

Fibrin gel is a surgical hemostatic agent derived from plasma coagulation proteins. Fibrin sealants are widely used to control bleeding in a range of surgical settings, and their use has increased due to the advent of minimally invasive surgical procedures that require meticulous hemostasis for suitable visualization of the surgical field. Fibrin sealants can be used for hemostasis, wound closure, and tissue sealing and have been recommended as the agents that come closest to the ideal operative sealant. Unlike synthetic adhesives, fibrin sealants have the advantage of being biocompatible and biodegradable, and are not associated with inflammation, foreign body reactions, tissue necrosis or extensive fibrosis. Resorption of the fibrin clot is achieved during normal wound healing within days to weeks after application, depending on the type of surgery, the proteolytic activity of the treated site and the amount of sealant used.

Fibrin sealants are usually derived from plasma proteins and contain two main components: fibrinogen and thrombin. When fibrinogen and thrombin are mixed, the fibrinogenic component is converted to fibrin monomers. Polymerization of fibrin monomers results in the formation of a semi-rigid fibrin clot that is able to interact covalently and non-covalently with tissue structures. The clot can be further stabilized by means of cross-linking of the alpha and gamma chains of fibrin in a reaction catalyzed by activated factor XIII. This cross-linking stimulates fibroblast adherence and promotes normal fibroblast growth in the clot. By mimicking the later steps of the physiological coagulation system, these processes allow fibrin sealants to stop blood loss and aid the wound healing process.

The term "fibrinogen", also known as "coagulation factor I", refers herein to a fibrous, adhesive glycoprotein found in blood plasma in an amount of about 200 to 400 mg/dL, and it plays an important role in all phases of hemostasis. Fibrinogen is made up of three pairs of chains: two Aα, two Bβ and two γ, which are coiled to form an alpha helix. The tertiary structure of fibrinogen is represented by three domains: a central node or E domain, made up of the N-terminal ends of the chains, and two side nodes or D domains, formed by the C-terminal ends. The fibrinopeptides A and B located in the central E domain are rich in glutamic acid and aspartic acid residues that confer a high density of negative charges that induce repulsion between adjacent fibrinogen molecules. The formation of stable fibrin depends on the ability of thrombin to cleave the Arg16-Gly17 and Arg14-Gly15 bonds at the N-terminal ends of the Aα and Bβ chains of fibrinogen and release fibrinopeptides A and B, respectively. This causes the loss of the negative charges present in the E domains and favors the formation of fibrin monomers that associate with each other by weak electrostatic bonds to form fibrin polymers, which are stabilized by activated FXIII.

The term "blood", as used herein, refers to a tissue fluid that flows through the capillaries, veins and arteries of all vertebrates. Its characteristic red color is due to the presence of the red pigment content of erythrocytes. It is a specialized type of connective tissue with a liquid colloidal matrix and complex constitution. In fact, blood comprises a solid phase formed by cells (including leukocytes, erythrocytes and platelets) and a liquid phase, represented by plasma. Its main function is logistic distribution and systemic integration, the containment of which in vessels (vascular space) supports its distribution (blood circulation) throughout most of the organism.

The term "erythrocytes" or "red blood cells", in the sense used in the present description, refers to the blood corpuscles that will transport oxygen and carbon dioxide in blood. Corpuscles are known to lack nuclei and organelles (only in mammals), so they cannot strictly be considered cells. They contain some enzymatic pathways, and their cytoplasm is almost entirely occupied by hemoglobin, a protein responsible for oxygen transport. The plasma membrane of erythrocytes contains glycoproteins (DCs) that define the different blood groups and other cell identifiers. Erythrocytes are disc-shaped, biconcave, depressed in the center; this increases the effective surface area of the membrane. Mature erythrocytes lack a nucleus, because it is dislodged in the last stage of maturation in the bone marrow before entering the bloodstream.

The term "anticoagulated" is used herein to refer to blood that has been treated with an anticoagulant. In turn, the term "anticoagulant", in the sense used in the present description, refers to an endogenous or exogenous substance that interferes with or inhibits blood coagulation. Non-limiting examples of anticoagulants are antithrombin III activators and heparin, low molecular weight ardeparin, certoparin, nadroparin, logiparin, parnaparin, reviparin and tedelparin, and recombinant antithrombin III, tissue factor (TF)-factor Vila inhibitor, such as TF mutants, anti-TF antibodies, factor VII inhibitors, thrombin inhibitors such as anti-statin factor Xa inhibitors, TAP (tick anticoagulant peptide), DX9065, lefaxin, fondaparinux, terostatin, YM-75466 and ZK-807,834, antibodies against factor IXa and Xa, protein C pathway activators and selective thrombin inhibitors such as argatroban, bivalirudin, efegatran, H376/95, hirugen, inogatran, melagatran, napsagatran, UK-156406 and ximelagatran, and chemical compounds that capture calcium ions, such as ethylenediaminetetraacetic acid (EDTA), citrate (in general, sodium citrate) and oxalate.

In a particular embodiment, the anticoagulant is sodium citrate.

In a particular embodiment, the anticoagulant, preferably sodium citrate, is comprised in a solution, preferably an aqueous solution. In a particular embodiment, the sodium citrate is comprised in a solution, preferably an aqueous solution, comprising citric acid.

In another embodiment, the anticoagulant, preferably sodium citrate, is present at a concentration of between 1% and 10%, between 2% and 8%, between 2% and 6%, between 3% and 5%, between 3% and 4%, more specifically at a concentration of 3.8% (w/v), w/v in reference to the weight of anticoagulant with respect to the volume of the solution.

The term "subject", as used herein, refers to any animal with blood circulation, preferably a mammal, more preferably a primate, and even more preferably, a human being, male or female, of any age or race.

In the present invention, the term "plasma" or the expression "blood plasma" refers to the non-cellular fluid portion of the blood of humans or animals prior to coagulation. It is made up of 90% water, 7% protein and the remaining 3% fat, glucose, vitamins, hormones, oxygen, carbon dioxide and nitrogen, in addition to metabolic waste products such as uric acid. It is the main component of blood, accounting for about 55% of the total blood volume, while the remaining 45% corresponds to formed elements. The viscosity of blood plasma is 1.5 times that of water. The main plasma proteins are as follows: albumin, which also participates in lipid transport; globulins, which are mainly related to the body's defense mechanisms (for example immunoglobulins) or to iron transport (transferrin); fibrinogen, a protein that is essential for blood coagulation; prothrombin, a protein that participates in the coagulation process; and low- and high-density lipoproteins (LDL and HDL, respectively), which participate in cholesterol transport. The set of plasma proteins, also called "plasma factors", performs, among other functions, those of:
- maintaining plasma volume and blood volume,
- protecting and maintaining the pH stability of the blood,
- participating in blood viscosity, and therefore contributing minimally to peripheral vascular resistance and vascular pressure (blood pressure), and
- intervening in the electrochemical equilibrium ion concentration (the so-called Donnan effect).

The first composition of the method of the invention is obtained from a platelet-poor blood plasma (PPP). The expression "platelet-poor plasma" or "PPP", in the present invention, refers to a plasma fraction, i.e., the non-cellular fluid portion of the blood of humans or animals prior to coagulation, which remains after obtaining and separating a platelet concentrate or "cPLT" from a blood sample. Plasma would contain proteins, electrolytes, antibodies, antigens, hormones, growth factors and fibrinogen. PPP may have high levels of fibrinogen, which has the ability to form a fibrin-rich clot once activated.

In a particular embodiment, the PPP from which the first composition of the method of the invention comprising the fibrinogenic concentrate is obtained contains a platelet concentration of between 1x10² and 1x10⁵, between 1x10³ and 1x10⁵, more particularly between 1x10⁴ and 1x10⁵ platelets/µL. In another particular embodiment, the platelet concentration is less than 1 x 10⁵ platelets/µL. In another particular embodiment, the platelet concentration is less than 1x10⁴ platelets/µL.

The term "platelets", as used herein, refers to small (2-3 micrometers in diameter), oval, cell units without a nucleus generated under normal conditions in bone marrow from the cytoplasmic fragmentation of megakaryocytes. Platelets circulate in the blood of all mammals and participate in hemostasis, participating in the formation of blood clots. Platelets release a large number of growth factors and/or cytokines, including platelet-derived growth factor (PDGF, also abbreviated as PDGFAA), a potent chemotactic agent, and transforming growth factor beta (TGF-β1), which stimulates extracellular matrix formation. Depending on certain conditions, platelets also synthesize many other molecules, as discussed above, essential to the inflammatory process and to the tissue repair and regeneration process. These growth factors or molecules have been shown to play an important role in connective tissue repair and regeneration. Other proteins and growth factors produced by platelets and associated inflammatory, repair and regeneration processes include basic fibroblast growth factor (BFGF), insulin-like growth factor 1 (IGF-1), insulin-like growth factor 2 (IGF-2), epithelial growth factor (EGF), hepatocyte growth factor (HGF), vascular endothelial growth factor (VEGF), interleukin-8, keratinocyte growth factor, connective tissue growth factor. The local application of all these growth factors, molecules and proteins produced by platelets participates in and accelerates the healing process of different injuries. Platelets also release large amounts of thrombin and adenosine diphosphate ADP, calcium, thromboxane A2, PF4, PAI-1, fibrinogen, fibronectin, thrombomodulin, FV, FXIII, RANTES, thrombospondin, WWF PF-3, serotonin, hydrolytic enzymes, etc.

In one embodiment, the blood plasma (PPP) is obtained by means of centrifugation of the anticoagulated blood sample at a speed of at least 1300 g, preferably at least 1500 g, particularly at a speed of between 1300 and 2500 g, between 1300 and 2200 g, between 1300 and 2000 g, between 1300 and 1800 g, between 1400 and 1600 g. In one embodiment, the blood plasma (PPP) is obtained by means of centrifugation of the anticoagulated blood sample for at least: 10 minutes, 11 minutes, 12 minutes, 13 minutes, 14 minutes, 15 minutes, 16 minutes, 17 minutes, 18 minutes, 19 minutes or 20 minutes, preferably at least 15 minutes; particularly up to 45 minutes, 40 minutes, 35 minutes, 30 minutes or 25 minutes. In another embodiment, these speeds and times are combined. One skilled in the art will know how to combine and change the conditions of centrifugation speed and centrifugation time by modifying them in a compensatory manner, i.e., by increasing the centrifugation force and decreasing the centrifugation time or alternatively by decreasing the centrifugation speed and increasing the time to obtain the PPP.

In a more particular embodiment, the PPP is obtained by means of centrifugation of the anticoagulated blood sample at a speed of at least 1300 g for at least 10 minutes. In another particular embodiment, the PPP is obtained by means of the centrifugation of anticoagulated blood at a speed of at least 1500 g for at least 15 minutes. The upper limits of these values can be those indicated above.

After obtaining the PPP, for example after separating it from the other blood components by centrifugation as previously indicated, said PPP is collected by means of methods common to one skilled in the art.

In the context of the present invention, the fibrinogenic concentrate can be isolated from the PPP by means of different methodologies known in the state of the art for precipitating proteins and subsequently dissolving said proteins.

The precipitation of proteins from the PPP can be performed, for example, by means of salt precipitation or by means of alcohol precipitation.

In a particular embodiment, the precipitation is performed by means of salt precipitation. Said precipitation consists of adding salt ions to the solution to cause the restriction of the water molecules available to the proteins, leading to the destruction of the hydrogen bonds. The interaction between the proteins is stronger than between the protein and the available water molecules, leading to protein aggregation and precipitation. Examples of salts used are zinc sulfate and ammonium sulfate.

In another preferred embodiment, precipitation is performed by using an alcohol solution. The addition of alcohol to the solution reduces protein hydration by removing surrounding water and proteins, leading to aggregation and precipitation. Examples of alcohols for protein precipitation include, without limitation, an alkanol, such as ethanol, methanol and isopropanol, preferably ethanol. The alcohol solution is preferably added in a volume of between 5% and 15%, more preferably in a volume of about 10%, with respect to the volume of the PPP. In one embodiment, the alcohol solution comprises at least 70%, preferably at least 90%, such as 96%, by volume of alcohol with respect to the total volume of the solution.

In a particular embodiment, the fibrinogenic concentrate is obtained from the PPP by means of alcohol precipitation and subsequent dissolving of the precipitate obtained. In a more particular embodiment, the fibrinogenic concentrate is obtained from the PPP by means of precipitation with an ethanol solution and subsequent dissolving of the precipitate obtained.

Optionally, the mixture is cooled during precipitation, preferably at a temperature of between 2°C and 8°C, more preferably at a temperature of about 4°C. In one embodiment, this temperature is maintained for at least: 10 minutes, 15 minutes, 20 minutes, 25 minutes, 30 minutes, preferably at least 30 minutes, in one embodiment up to 120 minutes, 90 minutes or 60 minutes.

The proteins are subsequently isolated from the optionally cooled precipitated mixture, for example, the optionally cooled precipitated mixture can be subjected to an additional centrifugation. In a particular embodiment, the additional centrifugation is performed at a speed of at least 300 g, such as between 300 g and 900 g, for example at 580 g. In a particular embodiment, the additional centrifugation is performed for at least 5 minutes, such as between 5 and 20 minutes, or between 6 and 10 minutes, for example for 8 minutes. In a particular embodiment, these speeds and times are combined. After the additional centrifugation, the supernatant is separated and the precipitate formed (usually in pellet form) is preserved.

Finally, the isolated precipitate is dissolved by means of heating and/or by addition of an isotonic solution, preferably by means of heating. Preferably, heating is performed at a temperature of at least 30°C, such as between 30°C and 44°C, preferably between 34°C and 40°C, for example at about 37°C.

In another embodiment, dissolving is performed by heating at the temperature of the subsequent mixing of the first composition with the second composition, i.e., at the temperature of step f) of the first aspect of the invention.

In a preferred embodiment, the temperature of the resulting solution, first composition of the method of the invention, is maintained at the heating temperature until being mixed with the second composition of the method of the invention.

In a particular embodiment, the first composition comprising the fibrinogenic concentrate is obtained from blood plasma (PPP) by means of a method comprising the following steps:
a) precipitating the proteins from the plasma, particularly as previously indicated;
b) optionally cooling during the precipitation of step a), particularly as previously indicated;
c) isolating the precipitate obtained in a) or b), particularly as previously indicated; and
d) dissolving said precipitate, particularly as previously indicated.

In a more particular embodiment, the method for obtaining a fibrinogenic concentrate from PPP comprises the following steps:
a) adding an alcohol solution, preferably an ethanol solution, to the plasma, preferably in a volume of between 5% and 15%, more preferably in a volume of about 10%, with respect to the volume of said plasma;
b) cooling the mixture obtained in a) at a temperature of between 2°C and 8°C, for example at a temperature of about 4°C;
c) isolating the precipitate obtained in b) by means of centrifugation at a speed of at least 580 g for at least 8 minutes;
d) dissolving the isolated precipitate by heating, preferably at a temperature of between 30°C and 44°C, for example at about 37°C.

In a particular case of the preceding embodiment, step d) of dissolving the precipitate, dissolving is performed by heating at a temperature of between 34°C a 40°C; in another particular case, in said step d) of dissolving the precipitate, dissolving is performed by heating at the temperature of the subsequent mixing of the first composition with the second composition.

In a particular embodiment, the fibrinogen concentration in the first composition of the invention is between 6 and 9 times greater than the fibrinogen concentration in blood plasma. In a particular embodiment, the fibrinogen concentration in the first composition of the invention is between 8 and 80 mg/ml, more specifically between 10 and 70 mg/ml, between 12 and 60 mg/ml, between 12 and 50 mg/ml, between 12 and 40 mg/ml, preferably between 12 and 36 mg/ml.

In another particular embodiment, the platelet concentration of the first composition of the invention is less than 1 x 10⁵ platelets/µl, 2 x 10⁵ platelets/µl, 3 x 10⁵ platelets/µl, 4 x 10⁵ platelets/µl, 5 x 10⁵ platelets/µl, 6 x 10⁵ platelets/µl, 7 x 10⁵ platelets/µl, 8 x 10⁵ platelets/µl and 9 x 10⁵ platelets/µl, more preferably less than 3.5 x10⁵ platelets/µl.

The second composition of the method of the invention is obtained from a platelet-rich plasma. As used in the present specification, the expression "platelet-rich plasma" or "PRP" is understood to refer to any plasma preparation having a higher platelet density, more preferably twice the platelet density, and still more preferably four times the platelet density, compared to the blood sample from which the plasma has been derived. In one embodiment, the platelet-rich plasma is characterized by a higher platelet concentration, for example two, three or four times higher, than that observed in the blood sample from which the plasma has been derived, which is between 150,000 and 350,000 platelets per microliter of blood (pl/µl). In that sense, in a particular embodiment the level of platelets in the PRP is between 300,000 and 700,000 pl/µl, between 400,000 and 900,000 pl/µl, between 600,000 and 1,200,000 pl/µl.

Platelet-rich plasma is plasma containing concentrated platelets and can be prepared from whole blood using a range of methods known in the art. For example, the platelet-rich plasma that is useful in the practice of the invention can be prepared by subjecting whole blood to centrifugation to separate the platelet-rich plasma from the red blood cells. The platelet-rich plasma fraction is then subjected to a second round of centrifugation to produce a platelet pellet and a platelet-poor plasma supernatant. Most of the platelet-poor plasma can be removed, leaving concentrated platelets and a small proportion of platelet-poor plasma.

In a particular embodiment, the preparation of PRP is a method comprising:
I) treating a blood sample from a subject with anticoagulant, wherein the meaning of anticoagulant is the same as provided above;
II) centrifuging the sample; and
III) obtaining the supernatant, the supernatant being PRP.

In one embodiment, the centrifugation of the anticoagulated blood sample is performed at a speed of at least 150 g, at least 300 g, at least 350 g, at least 400 g, at least 450 g, at least 500 g, at least 550 g, at least 600 g, at least 650 g, at least 700 g, at least 750 g, at least 800 g, at least 850 g, at least 900 g, at least 950 g, or at least 1000 g, and preferably at least 580 g, and preferably at a speed of up to 1100 g, 1400 g, 1500 g, 1600 g, 1700g, 1800g, 1900 g or 2000 g. In one embodiment, the centrifugation of the anticoagulated blood sample is performed for at least 2 minutes, at least 3 minutes, at least 4 minutes, at least 5 minutes, at least 6 minutes, at least 7 minutes, at least 8 minutes, at least 9 minutes, at least 10 minutes, at least 11 minutes, at least 12 minutes, at least 13 minutes, at least 14 minutes, at least 15 minutes, at least 16 minutes, at least 17 minutes, at least 18 minutes, at least 19 minutes or at least 20 minutes, preferably for at least 8 minutes, particularly up to 45 minutes, 40 minutes, 35 minutes, 30 minutes or 25 minutes. In another embodiment, these speeds and times are combined.

In another particular embodiment, the centrifugation of the anticoagulated blood sample is performed at a speed of at least 150 g, at least 300 g, at least 350 g, at least 400 g, at least 450 g, at least 500 g, at least 550 g, at least 600 g, at least 650 g, at least 700 g, at least 750 g, at least 800 g, at least 850 g, at least 900 g, at least 950 g, or at least 1000 g, and preferably at least 580 g, and preferably at a speed of up to 1400 g, and for at least 2 minutes, at least 3 minutes, at least 4 minutes, at least 5 minutes, at least 6 minutes, at least 7 minutes, at least 8 minutes, at least 9 minutes, at least 10 minutes, at least 11 minutes, at least 12 minutes, at least 13 minutes, particularly up to 14 minutes. As known to one skilled in the art, the conditions for obtaining PRP can be various and, according to common general knowledge, conditions of centrifugation speed and time can be combined, such that the centrifugation speed increases and the centrifugation time decreases or vice versa in a compensatory manner for obtaining the PRP. One skilled in the art will know how to combine said variables to obtain the desired product.

In a particular embodiment, the PRP from which the second composition of the method of the invention comprising thrombin and growth factors is obtained, is obtained by means of centrifugation of the anticoagulated blood sample at a speed of at least 580 g for at least 8 minutes. In a more particular embodiment, the PRP is obtained by means of centrifugation of the anticoagulated blood sample at a speed of between 580 g and 1400 g for between 8 and 14 minutes.

In the present invention, the term "thrombin" refers to a glycoprotein enzyme that is not a normal constituent of circulating blood, but rather is generated from prothrombin and factor II in the presence of calcium ions and by means of a reaction catalyzed by activated factor Xa, in the final step of the two converging cascades of reactions, the intrinsic pathway and the extrinsic pathway of coagulation. Thrombin activates the degradation of fibrinogen into fibrin monomers, in addition to activating factor XIII or fibrin stabilizing factor. Other important biological functions of thrombin include: 1) activation of platelets to release components (cytokines) from their α-granules and dense granules; 2) potentiation of the synthesis, release and expression of proteins in endothelial cells (angiogenesis); and 3) potent induction of chemotaxis of neutrophils (inflammatory factors) and macrophages (cytokine production).

The expression "growth factor" as used in the present invention refers to intercellular mediators, including, among others, growth factors, cytokines and chemokines stored in the cytoplasm of platelets and released after platelet aggregation or clot formation. This secretion is intense in the first few hours and platelets continue to synthesize these mediators from their mRNA stores for at least another 7 days. More than 800 different proteins are secreted into the surrounding media, which have a paracrine effect on different cell types: myocytes, tendon cells, mesenchymal stem cells of different origins, chondrocytes, osteoblasts, fibroblasts and endothelial cells. Cell proliferation, angiogenesis and cell migration are stimulated, resulting in tissue regeneration. There are also reports confirming that platelets secrete antimicrobial peptides, suggesting an antibiotic effect.

In particular, mediators would include, without being limited to, growth factors, pro-inflammatory cytokines, anti-inflammatory cytokines and chemokines. Specific examples of said mediators include, without being limited to, platelet-derived growth factor (PDGF), endothelial growth factor (VEGF) and transforming growth factor (TGF), fibroblast growth factor (FGF), along with the anti-inflammatory and pro-inflammatory cytokines interleukin (IL)-4, IL-8, IL-13, IL-17, IL-10, tumor necrosis factor (TNF)-α and interferon (IFN)-α, without being limited to same. Likewise, said intercellular mediators also include anti-inflammatory and analgesic compounds, in part due to the presence in platelets of high amounts of the chemokine RANTES (CCL5).

Therefore, in the sense of the present invention, the expression "growth factor" encompasses all other molecules, such as signaling proteins, peptides, glycoproteins, mucoproteins or hormones which are not considered cytokines, but which exercise a signaling function between cells.

One of the most notable growth factors produced by platelets would be platelet-derived growth factor (PDGF), which is secreted by platelets during the early stages of wound and fracture healing.

PDGF includes both PDGF-A (Genbank accession no. NP_002598) and PDGF-B (Genbank accession no. NP_002599) which can be homodimerized or heterodimerized to produce three different isoforms: PDGF-AA, PDGF-AB or PDGF-BB. PDGF-A is only able to bind to PDGF receptor α (PDGFR-α, including PDGFR-α/α homodimers). PDGF-B can bind to both PDGFR-α and to a second PDGF receptor (PDGFR-β). More specifically, PDGF-B can bind to PDGFR-α/α and PDGFR-β/β homodimers, as well as to PDGFR-α/β heterodimers.

The expression "platelet-free" is used herein to include plasmas and compositions which are completely devoid of these materials. In the context of the present invention, platelet activation has triggered the coagulation cascade and this causes the platelets to rupture, releasing their entire contents into the medium, whereby the serum to be drawn, comprising thrombin and growth factors, would be platelet-free.

The expression "platelet activation" refers herein to the process whereby platelets present in plasma aggregate to give rise to clot formation and secretion of growth factors or intercellular mediators from their cytoplasmic granules. Platelets can be activated by a large number of substances to which they react within a few seconds. When exposed to these agonists, platelets exhibit different responses, which are closely associated *in vivo,* but which, *in vitro,* can be studied separately: adhesion and shape change, aggregation, secretion and expression of procoagulant activity.

Activated platelets change their shape, becoming more spherical, and forming pseudopods on their surface. In this way they take on a star shape. After activation, secretion of granules occurs. Platelets contain alpha granules and dense granules. Activated platelets excrete the contents of these granules into their canalicular systems and into the surrounding blood. There are two types of granules: (i) dense granules (containing ADP or ATP, calcium, and serotonin) and (ii) α granules (containing platelet factor 4, transforming growth factor beta 1 (TGF beta 1), platelet-derived growth factor, fibronectin, B-thromboglobulin, vWF, fibrinogen, and coagulation factors (factors V and VIII), among others.

In a particular embodiment, the platelets are activated by a substance selected from calcium chloride, thrombin and collagen, preferably calcium chloride. Platelet activation is carried out by means of the addition of that substance to the PRP. In a more preferred embodiment, platelet activation is carried out by means of the addition of a calcium chloride solution, preferably an aqueous calcium chloride solution, of at least 5%, such as 5 to 30%, more preferably 10% by weight of calcium chloride in relation to the volume of the solution (w/v). The calcium chloride solution is preferably added in a volume of between 0.05% and 1%, more preferably in a volume of about 0.2%, with respect to the volume of the PRP.

In another embodiment, the platelets are activated mechanically by means of contact with glass microspheres.

Platelet activation triggers a coagulation process, and after removal of the resulting clot, a solution (a serum) which constitutes the second composition of the invention is obtained. This composition comprises thrombin and growth factors and is platelet-free.

The clot can be removed mechanically by means of any instrument useful for this purpose, such as for example forceps. Alternatively, the serum obtained after platelet activation can be isolated by absorption by means of a pipette or syringe suitable for this purpose.

In one embodiment, the first and/or second composition is heated at the mixing temperature of step f) before subjecting same to said step f).

The term "centrifugation" refers to a method whereby solids of different densities are separated from liquids through a rotational force, which applies a centrifugal force greater than that of gravity to the mixture. The solids and particles of higher density settle out. Examples of centrifugation include, without limitation, differential centrifugation, isopycnic centrifugation, zonal centrifugation or ultracentrifugation. In the context of the present invention, differential centrifugation is preferred.

Centrifuges used in the context of the present invention can be obtained from a range of sources, including, for example, the PLACONTM centrifuge, which can be obtained from OCT USA Inc, Torrence.

In a particular embodiment, the blood sample from which the first composition is obtained and the blood sample from which the second composition is obtained come from the same subject.

In a particular embodiment, the blood sample from which the first composition is obtained and the blood sample from which the second composition is obtained come from different blood draws from the same subject.

In another particular embodiment, the blood sample from which the first composition is obtained and the blood sample from which the second composition is obtained come from the same draw. In a particular manner, when the first and second compositions of the method of the invention are obtained from the same draw, then the first composition is obtained from a volume of between 60% and 80% of the total blood sample and the second composition is obtained from a volume of between 20% and 40% of the drawn blood sample. In another embodiment, the first composition is obtained from a volume of 80% of the drawn blood and the second composition is obtained from 20% of the remaining blood volume.

In another particular embodiment, the first and second compositions of the method of the invention are mixed in a volumetric ratio of 5:1 to 1:5, more specifically at a volumetric ratio of 1:1.

In one embodiment, the first and second compositions are heated separately at the mixing temperature of step f) and this temperature is maintained until the mixing of step f).

### Gel of the invention

In a second aspect, the invention relates to a fibrin gel obtained by means of the method of the first aspect of the invention.

In a particular embodiment, the fibrin gel has a surface adhesion strength of between 200 mN/cm² and 600 mN/cm² measured in a uniaxial testing machine. Said universal testing machine is capable of precisely controlling both the indenter displacement and the applied force. More particularly, the equipment used in the measurement is a Zwick/Roell uniaxial testing machine ZwickiLine Z1.0 model (sn: 734188-2019). The load cell used was a Zwick/Roell Xforce P with a maximum load of 50 N (sn: 781140). The software to control the machine and record both load and displacement was Zwich/Roell testXpert III v1.4 with all patches installed up to version sx406-218-5. The machine was calibrated *in situ* according to DIN standard EN ISO 9513:2013-05 (Deusche Akkreditierungsstelle GmbH laboratory, Germany).

In another particular embodiment, the gel of the second aspect of the invention has a Young's modulus of between 11 KPA and 16 KPA, an energy absorption capacity of between 4 and 4.5 mJ/m, and a swelling of between 1.1 and 1.63 measured as the hydrated to dehydrated gel weight ratio.

In said particular embodiment, Young's modulus and energy absorption measurements are performed by means of instrumented indentation tests with a spherical indenter. More specifically, the steel sphere used for the indentations is 5 millimeters in diameter and the indentations are made directly on P24 well plates where the tested materials are manufactured. The average number of tests on each well is 6. In particular for said embodiment, a universal testing machine capable of precisely controlling both the indenter displacement and the applied force was used. The equipment used is a Zwick/Roell uniaxial testing machine ZwickiLine Z1.0 model (sn: 734188-2019). The load cell used was a Zwick/Roell Xforce P with a maximum load of 50 N (sn: 781140). The software to control the machine and record both load and displacement was Zwich/Roell testXpert III v1.4 with all patches installed up to version sx406-218-5. At the time of performing the experiments, the machine was calibrated according to DIN standard EN ISO 9513:2013-05. The machine was calibrated *in situ* by the Deusche Akkreditierungsstelle GmbH laboratory in Germany.

In an additional embodiment, the percentage of coagulation of the fibrin gel is between 67% and 87% measured as the weight of the clot formed after the mixing of step f) over the combined weight of the first composition and the second composition before the mixing thereof. The weight of the clot can be measured, for example, five minutes after starting the mixing.

In another particular embodiment, the platelet concentration in the fibrin gel is less than 5 x 10⁴ platelets/µl, x 6 x 10⁴ platelets/µl, 7 x 10⁴ platelets/µl, 8 x 10⁴ platelets/µl, 9 x 10⁴ platelets/µl, 1 x 10⁵ platelets/µl, 2 x 10⁵ platelets/µl, 3 x 10⁵ platelets/µl, 4 x 10⁵ platelets/µl and 5 x 10⁵ platelets/µl, more preferably 1.075 x 10⁵ platelets/µl.

In one embodiment, the method of the invention does not comprise any lyophilization step.

### Medical uses

In a third aspect, the invention relates to the fibrin gel of the second aspect of the invention for use in medicine.

Alternatively, the invention relates to the use of the fibrin gel of the second aspect of the invention for the preparation of a medicinal product.

Moreover, the present invention provides methods for treatment and use of the fibrin gel of the invention for the treatment of injured or traumatized tissues, including cartilage and bone defects. The method for treatment described herein is advantageous because it requires minimal preparation for use by the physician.

There are multiple uses of fibrin gel. It can function as a scaffold and be used as an implant *per se,* to provide mechanical support to a defective or injured site *in situ* and/or to provide a matrix within which cells from the defective or injured site can proliferate and differentiate, for example, for cartilage repair.

Therefore, in another aspect, the invention relates to the fibrin gel of the second aspect of the invention for use in the treatment of osteochondral injuries, meniscal injuries or ulcers, particularly vascular ulcers.

Alternatively, the invention relates to a method for the treatment or prevention of osteochondral injuries, meniscal injuries or ulcers, particularly vascular ulcers, wherein the method comprises the administration of the fibrin gel according to the second aspect of the invention to a patient in need of said treatment.

Likewise, in an alternative manner, the invention relates to the use of the fibrin gel according to the second aspect of the invention in the preparation of a medicinal product for the treatment of osteochondral injuries, meniscal injuries or ulcers, particularly vascular ulcers.

Treatments or uses contemplate administration of a therapeutically effective amount of fibrin gel to a patient. The term "therapeutically effective amount" refers to the amount of fibrin gel sufficient to provide the desired effect, i.e., to achieve an appreciable prevention, cure, delay, reduction in severity or improvement of one or more symptoms arising from a disease, and will generally be determined by, among other things, the characteristics of the agent itself and the therapeutic effect to be achieved. It will also depend on the subject to be treated, the severity of the disease suffered by said subject, the pharmaceutical form chosen, etc. For this reason, one skilled in the art will have to adjust the dose based on the above mentioned variables. In one embodiment, the effective amount causes the improvement of one or more symptoms of the disease being treated.

As used herein, "osteochondral injury" or "cartilage injury" is that which occurs in the osteochondral unit of the joint, the tissue comprising both bone and cartilage that encases the bone in joints and facilitates joint engagement and protects the joint from excessive friction.

As used herein, cartilage refers to a type of connective tissue containing chondrocytes included in an intercellular material (often referred to as the "cartilage matrix") comprising collagen fibrils (predominantly type II collagen along with other minor types, e.g., types IX and XI), different proteoglycans (e.g., chondroitin sulfate, keratan sulfate and dermatan sulfate proteoglycans), other proteins, and water. Cartilage, in the form used herein, includes articular and meniscus cartilage. Articular cartilage covers the surfaces of the bone portions of joints and allows joint movement without direct bone-to-bone contact, and thus prevents wear and damage to juxtaposed bone surfaces. The most typical healthy articular cartilage is also described as "hyaline", i.e., with a characteristic ground-glass appearance. Meniscal cartilage is usually found in joints that are exposed to both concussion and movement. These meniscal cartilage locations include the temporo-mandibular, sternoclavicular, acromioclavicular, wrist and knee joints.

Joints are one of the common means by which the bones of the skeleton are connected. The ends of normal articulating bones are covered with articular cartilage tissue, which allows the movement of bones with virtually no friction against each other.

Articular cartilage is characterized by a particular structural organization. It consists of specialized cells (chondrocytes) included in an intercellular material (often referred to in the literature as the "cartilage matrix"), rich in proteoglycans, predominantly type II collagen fibrils, other proteins and water. Cartilage tissue is not innervated or penetrated by the vascular or lymphatic systems. However, in the mature adult joint, the underlying subchondral bone tissue, which forms a narrow, continuous plate between bone tissue and cartilage, is innervated and vascularized. Below this bony plate, the bone tissue forms trabeculae, which contain the marrow. In immature joints, the articular cartilage has beneath it only trabeculae of primary bone. A portion of the meniscus tissue present in joints also consists of cartilage the replacement of which is similar to that of articular cartilage.

Two types of defects or injuries are recognized in cartilage tissue, i.e., full-thickness defects and surface defects. These defects differ not only in the degree of physical damage caused to the cartilage, but also in the nature of the repair response that each type of injury can induce.

Full-thickness defects extend into the subchondral bone and can cause severe pain since the bony plate contains nerve endings from sensory nerve centers. These defects generally arise from major trauma or during the later stages of a degenerative joint disease, such as osteoarthritis. Full-thickness defects can sometimes lead to hemorrhage and induction of a repair reaction from the subchondral bone. The repair tissue formed is a type of vascularized fibrous cartilage, with insufficient biomechanical properties, and does not persist in the long term.

Surface defects in articular cartilage tissue are restricted to the cartilage tissue itself. These defects are important because they do not heal and show no predisposition for repair reactions.

These defects may appear as fissures, turf formations or grooves on the cartilage surface, or they may present a "rough flesh" appearance in the affected tissue. They contain non-hemorrhagic vessels (blood spots) like those seen in full-thickness defects. Surface defects may have an unknown cause, but are often the result of mechanical disorders leading to wear of the cartilage tissue. Mechanical disorders may be caused by trauma to the joint, e.g., a displacement of torn meniscal tissue into the joint, meniscectomy, a joint laxity caused by a torn ligament, faulty joint alignment, or bone fracture, or by hereditary diseases. Surface defects are also characteristic of the early stages of degenerative joint diseases, such as osteoarthritis. Because cartilage tissue is not innervated or vascularized, surface defects are not painful. However, although painless, surface defects do not heal and often degenerate into full-thickness defects.

It is generally believed that because articular cartilage lacks vasculature, damaged cartilage tissue does not receive sufficient or appropriate stimuli to induce a repair response. It has been theorized that chondrocytes in cartilage tissue are generally not exposed to sufficient amounts of repair stimulating agents such as growth factors and fibrin clots, typically present in damaged vascularized tissue.

One experimental approach used to expose damaged cartilage tissue to repair stimuli involves drilling or shaving through the cartilage into the subchondral bone to cause bleeding [Buckwalter *et al.* (1990), *supra*]. Unfortunately, the tissue repair response to this surgical trauma is generally comparable to that observed to occur naturally in full-thickness defects that cause bleeding, namely, formation of a type of fibrous cartilage that exhibits insufficient biomechanical properties and does not persist over the long term.

Treatment of cartilage defects can be performed during a simple arthroscopic or surgical procedure, using the gel of the invention. According to some methods, after identification of the defect, the defect is treated by means of the steps of (1) filling the defect area with an enzyme, which degrades proteoglycans present on the surface of the defect, (2) removing the enzyme, and (3) recomposing the defect with a composition comprising the gel, a proliferation agent and a transformation factor in a suitable delivery system.

The fibrin gel can be used in conjunction with other therapeutic procedures, including chondral shaving, laser or abrasion chondroplasty, and drilling or microfracture techniques.

Cartilage defects or injuries in animals are readily identified visually during an arthroscopic examination of the joint, or during a simple examination of the injury or defect during open surgery. Cartilage defects can also be identified by inference using computer aided tomography (CAT) scanning, X-ray examination, magnetic resonance imaging (MRI), analysis of synovial fluid or serum markers, or by means of any other procedure known in the art.

Once the defect has been identified, the surgeon may choose to surgically modify the defect in order to increase the ability of the defect to physically retain the solutions and gel material described herein. Preferably, instead of presenting a flat or slightly concave geometry, the defect is either shaped to present vertical edges, or hollowed out to better retain the solutions and added material described herein.

In addition to the above mechanical measures, which will improve the adherence of the gel to the defect site, chemical measures can also increase the adhesion of the gel. These measures include degrading the surface layers of cartilage proteoglycans located on the surface of the defect to expose the collagen fibrils of the cartilage so that they can interact with the fibrin fibrils. Proteoglycans on the cartilage surface not only tend to interfere with the adherence of a fibrin matrix to the cartilage, but also locally inhibit thrombin activity.

In addition, matrix adhesion to the cartilage of the defect can also be increased by using fibrin glue (i.e., blood factor XIII or fibrin stabilizing factor) to promote the formation of chemical bonds (cross-linking) of the gel fibrils to the collagen fibrils of the cartilage located on the surface of the defect. The enzyme transglutaminase can be used to achieve the same effect. Other compounds capable of promoting adhesion of extracellular materials can also be used.

In one method, the surface of the defect is dried by soaking the area with a sterilized absorbent tissue, and the volume of the defect is filled with a sterilized enzyme solution for a period of 2-10 minutes to degrade the proteoglycans present on the cartilage surface. To degrade the proteoglycans, different enzymes can be used, separately or in combination, in buffered and sterilized aqueous solutions. The pH of the solution must be adjusted to optimize enzyme activity.

Enzymes useful for degrading proteoglycans in the methods of this invention include chondroitinase ABC, chondroitinase AC, hyaluronidase, pepsin, trypsin, chymotrypsin, papain, pronase, stromelysin and Staph V8 protease. The suitable concentration of a particular enzyme or combination of enzymes will depend on the activity of the enzyme solution.

The amount of time the enzyme solution is applied should be kept to a minimum so that proteoglycan degradation takes place predominantly in the area of repair. The total time of the procedure should be kept to a minimum, especially during an open arthrotomy, because cartilage can be damaged by exposure to air. For these reasons, in embodiments of the methods that include the step of degrading the proteoglycans by means of enzymatic digestion, digestion times of less than 10 minutes are preferred and digestion times of less than 5 minutes are highly preferred.

After the enzyme has degraded the proteoglycans on the surface of the defect, the enzyme solution should be removed from the defect area. Removal of the enzyme solution should be performed using an aspirator equipped with a fine suction tip and then soaked up with cotton material. Alternatively, the enzyme solution can be separated by soaking it up with cotton material alone.

After removal of the enzyme solution, the defect should be carefully rinsed, preferably three times, with sterilized physiological saline solution (e.g., 0.15 M NaCl). The rinsed defect site should then be dried. Sterile cotton material or gauze can be used to dry the defect site.

Alternatively, or in addition to the step of enzymatic treatment, the defect site can be recomposed with a compound, such as fibrin glue or transglutaminase, to increase adhesion of the matrix to the defect site. In a preferred embodiment, fibrin glue or transglutaminase is applied to the defect site after the defect site has been rinsed and dried after enzymatic treatment.

The defect site is then recomposed with the gel of the invention described herein, to fill the defect preferably up to its edges with the composition of the matrix, so that a flat surface is formed.

In a particular embodiment, the fibrin gel of the invention is injected at the site of the injury during the polymerization process, i.e., once the components thereof have been mixed on the outside of the injury, still in liquid form and in the moments prior to gel formation. In that sense, polymerization is completed at the site of the injury, adapting to the specific defect. Since, as indicated, clot formation after the mixing of composition 1 and composition 2 occurs in the next 1 or 2 minutes, the application of the composition to the site of the injury has to be performed as soon as possible once the mixing of the compositions occurs.

The sealant can be applied with a needle, as a spray or using other devices.

After the defect site is recomposed with the gel, the incisions made in the joint capsule and skin should be closed and the arthroscopy or open surgery completed.

The methods described herein can also be used to repair meniscal injury defects.

The term "meniscal injury" refers to an injury to the meniscus of the knee, the anatomical structure that is located intra-articularly in the knee and facilitates stability and cushioning of the joint.

The meniscus is made up of fibrocartilaginous tissue, composed mainly of type I collagen fibers (more than 90%), which is frequently damaged, affecting the stability and lubrication of the knee. Like in cartilage, the repair process of the meniscus is determined by its tissue characteristics, such as its abundant extracellular matrix (between 60 and 70% of the tissue weight) in which cells, i.e., fibrochondrocytes, fibroblasts and surface cells, are dispersed. Furthermore, its scarce vascularization is limited to 10-30% of its outer portion or meniscal wall, which also receives nerve endings and presents the highest cellularity.

Meniscal injuries compromise joint functions, since this structure provides stability to the knee and supports compressive stresses as well as tensile and shear forces. The meniscus also absorbs part of the mechanical stress on the joint and participates in the lubrication of the knee with the synovial membrane. Due to its functional importance in the knee and its vulnerability to repetitive injuries throughout a person's life, it is necessary to enhance its limited repair capacity for optimal recovery. In that sense, the fibrin gel can be applied as an adjuvant to surgical processes used for the repair of meniscal injuries such as meniscectomies and meniscal sutures.

In a particular embodiment, the fibrin gel of the invention is injected at the site of the injury during the polymerization process, i.e., once the components thereof have been mixed on the outside of the injury, still in liquid form and in the moments prior to gel formation. In that sense, polymerization is completed at the injection site. Since, as indicated, clot formation after the mixing of composition 1 and composition 2 occurs in the next 1 or 2 minutes, the application of the composition to the site of the injury has to be performed as soon as possible once the mixing of the compositions occurs.

The sealant can be applied with a needle, as a spray or using other devices.

After gel formation, the incisions made in the joint capsule and skin should be closed and the arthroscopy or open surgery completed.

In a particularly preferred embodiment, the fibrin gel for the medical uses indicated above is administered to the same patient from whom the blood samples, from which said fibrin gel, is obtained are drawn (autologous use).

In another particular embodiment, the fibrin gel for the medical uses indicated above is allogeneic, that is, it comes from blood samples from a donor from which the fibrin gel of the invention is obtained and it is administered to another subject who requires it.

The term "ulcer" refers to non-healing wounds, including neuropathic diabetic foot ulcers, traumatic wounds, arterial ulcers and ulcers of mixed etiology (arterial-diabetic, arterial-venous). An ulcer is a sore in the skin or mucous membrane, accompanied by tissue disintegration. Ulcers can result in complete loss of the epidermis and often portions of the dermis and even subcutaneous fat. Ulcers are most common on the skin of the lower limbs and in the gastrointestinal tract. An ulcer appearing on the skin often looks like inflamed tissue with an area of reddened skin. A skin ulcer is often visible in the case of exposure to heat or cold, irritation or a problem with blood circulation.

They may also be due to lack of mobility, which causes prolonged pressure on the tissues. This stress on blood circulation is transformed into a skin ulcer, commonly known as bedsores or pressure ulcers.[1] Ulcers often become infected and pus forms.

The term "vascular ulcers" refers to a poorly healing wound located on the leg. The cause is poor circulation due to pathological changes in arterial supply and/or venous drainage.

A venous ulcer is a complication of chronic venous insufficiency generally arising from changes secondary to deep or varicose thrombophlebitis of the superficial, perforating and/or deep veins. The venous system is made up of superficial and deep veins. Superficial veins are located between the skin and muscles, deep veins are located between muscles, and both types are connected to each other by perforating veins. All of these veins have valves that normally ensure that blood flows from the superficial veins to the deep system. If the valves are incompetent (varicose veins) or damaged (e.g., postphlebitic syndrome), venous insufficiency can occur allowing blood to back up and pool (build up) in the leg veins. This in turn increases blood pressure in the legs and causes leakage of fluid from the veins into the tissue with corresponding tissue edema. The edematous skin region may rupture and sometimes a visible skin ulcer may develop.

In another aspect of the invention, polymerization of the gel occurs at the site requiring treatment, for example at the injury or ulcer, i.e., the first and second compositions are added separately at the site to be treated and polymerization occurs directly at the site to be treated.

Therefore, the invention also relates to a method identical to the method of the first aspect of the invention, wherein step f) of mixing is performed *in vivo* instead of *in vitro.*

Likewise, the invention also relates to a combination of a first composition and a second composition as defined in the first aspect of the invention, for use in the medical uses indicated above, in particular wherein the use comprises mixing *in vivo,* particularly in the patient's body, more particularly on or at the site requiring treatment, for example the injury or ulcer to be treated, the first composition and the second composition preferably at a temperature of between 30°C and 44°C to form a fibrin gel. In this case, it is understood that the term combination refers to the whole of the first composition and the second composition prior to their mixing, since the mixing takes place subsequently *in vivo,* as already explained.

The obtaining of the first and second compositions is identical in all embodiments to that described in the first aspect of the invention.

Alternatively, the invention relates to a method of treating or preventing injuries or ulcers, in particular osteochondral injuries, meniscal injuries or ulcers, in particular vascular ulcers, wherein the method comprises the administration, to a patient in need of said treatment, of a first composition and a second composition as defined in the first aspect of the invention, wherein the method comprises mixing, on or at the injury or ulcer, the first composition and the second composition preferably at a temperature of between 30°C and 44°C to form a fibrin gel.

Likewise, alternatively, the invention relates to the use of a first composition and a second composition as defined in the first aspect of the invention in the preparation of a medicinal product for the medical uses indicated above, in particular for the treatment of osteochondral injuries, meniscal injuries or ulcers, in particular vascular ulcers, wherein the treatment comprises mixing, on or at the injury or ulcer, the first composition and the second composition preferably at a temperature of between 30°C and 44°C to form a fibrin gel.

Likewise, alternatively, the invention relates to a kit comprising a first composition and a second composition as defined in the first aspect of the invention, and instructions for mixing the first composition and the second composition preferably at a temperature of between 30°C and 44°C to form a fibrin gel.

In one embodiment, the mixing of the first and second compositions on or at the injury or ulcer, preferably at the ulcer, comprises combining the first and second compositions outside the human body, immediately thereafter, while that initial mixture still possesses a liquid consistency, applying that liquid mixture to the ulcer, where it will subsequently complete the polymerization (i.e., gel formation) process. In that sense, polymerization is completed at the site of the injury, adapting to the injury. Since, as indicated, clot formation after mixing of the first composition and the second composition occurs in the next 1 or 2 minutes, the application of the composition to the site of the injury or ulcer has to be performed as soon as possible after the compositions are combined.

The sealant can be applied with a needle, as a spray or using other devices.

After gel formation, conventional methods for ulcer protection such as gauze and dressings can be applied.

The invention will be described through the following examples, which must be taken as merely illustrative and non-limiting of the scope of the invention.

### EXAMPLES

### Technical description of the process

The following process was followed to develop this product:
A patient blood draw was performed using 9 mL volume draw tubes with 3.8% sodium citrate as anticoagulant. The number of tubes depends on the volume of product to be obtained (for example, 10 tubes to obtain a final product volume of 3-5 mL).

80% of the tubes (e.g., 8 of 10) were used for the concentration of the fibrinogen, i.e., to produce the first composition according to the invention. To that end:
1. The tubes were centrifuged at 1500 g for 15 minutes to obtain the plasma fraction of the blood (without red blood cells, white blood cells or platelets).
2. Once the plasma fractions were separated and collected in 9 mL tubes, a volume of 10% (0.9 mL) of ethanol 96% v/v (Reag. USP, Ph. Eur) was added, mixed well and cooled in an ice bath (2-8°C) for 30 minutes.
3. After 30 minutes, the tubes were centrifuged at 580 g for 8 minutes.
4. After this centrifugation, the supernatant was decanted, preserving the pellet/precipitate formed.
5. The tubes with the pellets/precipitates were subjected to a temperature of 37°C to dissolve them.
6. Once dissolved, they were pooled together in the same tube and conserved at 37°C until their use.

20% of the tubes (e.g., 2 of 10) were used to obtain the plasma solution of thrombin and growth factors, i.e., to produce the second composition according to the invention. To that end:
1. The tubes were centrifuged at 580 g for 8 minutes to separate the blood into its different fractions.
2. The entire plasma fraction was collected without including white blood cells or red blood cells, obtaining a platelet-rich plasma (PRP) with a platelet concentration similar to blood, type 11-00-11 or 22-00-11 according to the classification described in Kon E et al., 2020, (Expert Opin Biol Ther; 20(12):1447-1460).
3. The PRP from the tubes were pooled together in a 9 mL tube and 10% calcium chloride (2 µL per mL of PRP) was added to activate the platelets and trigger the coagulation process.
4. Once the clot was formed and retracted, the serum generated was collected and stored at 37°C until use.

Once the two compositions (fibrinogenic concentrate and plasma solution of thrombin and growth factors) were obtained, they were mixed in a v:v ratio of 1:1.

The fibrin gel gradually formed over the next 1-2 minutes after mixing.

### Technical analysis of the product obtained

### Fibrinogen concentration

In the assays performed, a fibrinogen concentration of between 6 and 9 times higher than the plasma concentration has been achieved by means of the described method.

### Clotting time

Formation occurs within the first two minutes after mixing the first composition and the second composition, having a readily applicable mixture that adheres to the tissue and remains consistent.

### Retraction

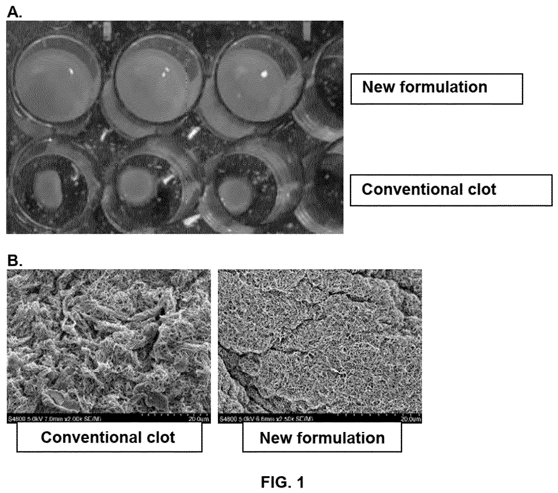

No retraction of PRP occurred over time compared to a conventional fibrin clot (Figure 1A). Lack of retraction was also observed in the fibrin fibers observed in electron microscopy (Figure 1B).

This lack of retraction allows for optimal and complete adaptation to the defect or injury over time.

### Adherence

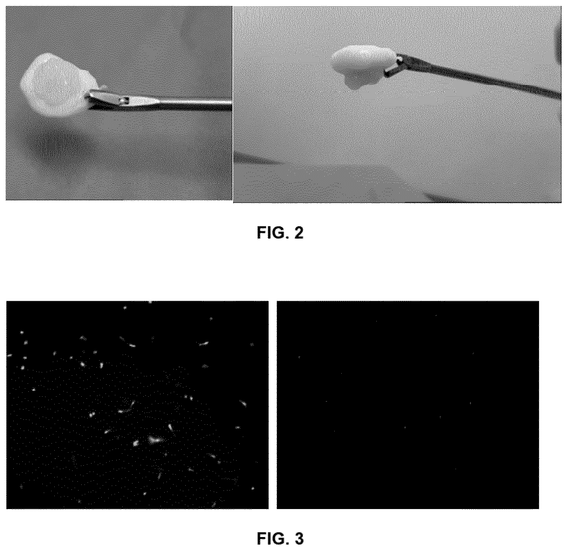

Mechanical adherence studies yielded a surface adhesion strength value of 402.2 ± 183.38 mN/cm² after five minutes of contact with the surfaces. Said adherence is transferred to the actual tissue (Figure 2), allowing the product to remain for a prolonged period of time and without danger of detachment.

### Biological studies

*In vitro* viability studies with calcein/ethidium show that the developed scaffold is not toxic to cells after experiments in cell cultures (Figure 3).

### Biomechanical studies

In biomechanical tests, the developed scaffold compared to the conventional scaffold shows a lower Young's modulus (less rigid) and also has a higher capacity to absorb energy.

| Formulation | | Initial thickness (mm) | Maximum displac. (mm) | Maximum force (mm) | Young's modulus (kPA) | Test energy (µJ) | Dissipated energy (mJ/m) |
|---|---|---|---|---|---|---|---|
| Conventional | Mean | 0.650 | 0.130 | 2.602 | 64.2 | 0.219 | 1.780 |
| | *Error* | *0.089* | *0.018* | *0.267* | *19.0* | *0.030* | *0.204* |
| New | Mean | 2.182 | 0.437 | 8.031 | 13.8 | 2.062 | 4.390 |
| | *Error* | *0.247* | *0.049* | *0.451* | *2.3* | *0.287* | *0.217* |

In swelling experiments, both formulations showed a ratio without significant differences (conventional: 1.49 ± 1.90 vs. new: 1.37 ± 0.26).

### Percentage of coagulation

The percentage of coagulation in the formation of the gel of the invention observed after mixing the first and second compositions was 77.03% ± 9.55 (p<0.0033), significantly higher than with other similar formulations.

The application of heat is important to obtain this value since the production of the fibrin gel by applying heat (37°C) generates a significantly higher percentage of coagulation than without heat (54.42% ± 14.32) (p<0.0022).

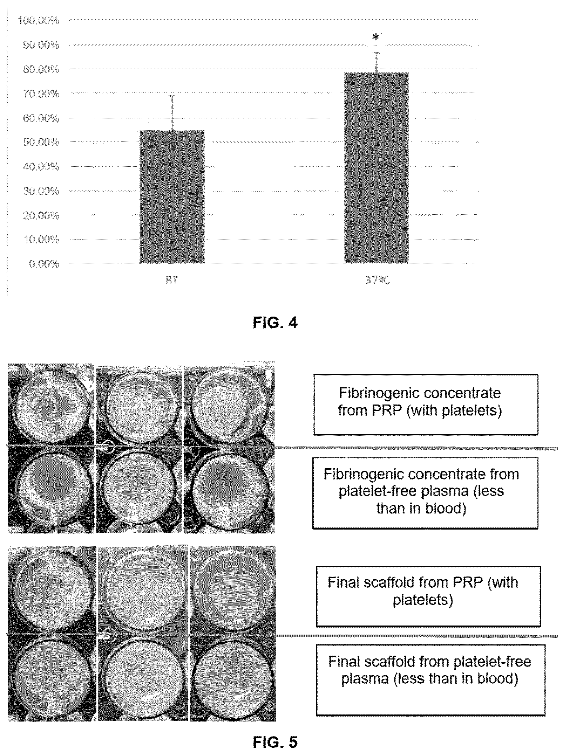

Therefore, the method of the invention allows better product handling and higher coagulation efficiency (Figure 4).

### Effect of the presence or absence of platelets in the gel

Depending on the centrifugation used on the blood to obtain the fibrinogenic concentrate, it may or may not present a high platelet concentration.

According to the process described in this protocol, the blood is centrifuged at 1500 g for 15 minutes to remove as many platelets as possible, unlike the protocols that start from a centrifugation to obtain a PRP. In that sense, the different platelet concentrations according to the different methods and times of the protocol would be as follows:

| Plasma fraction | | Fibrinogenic concentrate | | End product (scaffold) | |
|---|---|---|---|---|---|
| Without PLT | PRP | Without PLT | PRP | Without PLT | PRP |
| 61.4 ± 46.4 | 425.6 ± 103.5 | 214.8 ± 125.4 | 2089.2 ± 561.8 | 107.4 ± 62.7 | 1044 ± 280.9 |

| | | | | | |
|---|---|---|---|---|---|
| *Platelet concentration units: x10³ Plt*/*µL* | | | | | |

The excessive presence of platelets in the process yields a heterogeneous fibrinogenic concentrate and the presence of clots due to platelet aggregation, which impairs the fibrin gel formation and application process. On the contrary, in the fibrinogenic concentrate a concentration similar to or lower than the blood platelet concentration results in a uniform and homogeneous fibrinogenic concentrate that allows an optimal production and application process (Figure 5).

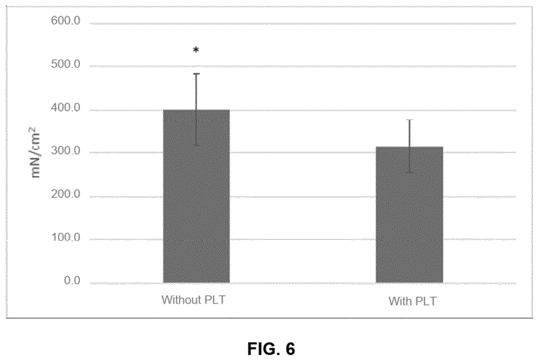

This inclusion of platelets in the formulation also affects other aforementioned properties such as adhesion, which is significantly lower when there is an excess of platelets in the formulation (Figure 6). The data obtained was as follows.

| Scaffold from concentrate without PLT | Scaffold from concentrate with PLT |
|---|---|
| 402.2 ± 183.38 mN/cm² | 317 ± 141.92 mN/cm² |

In summary, the main problems solved by eliminating the excessive platelet concentration is clot formation in the fibrinogenic concentrate, which hinders the formation of the end product and generates a heterogeneous scaffold. Moreover, this clot formation due to the presence of platelets seems to affect the adhesion capacity as suggested by the data relating to adhesion strength, which is significantly higher in the scaffold without platelets.

## Claims

1. An *in vitro* method for producing a fibrin gel comprising the following steps:
a) obtaining a platelet-poor plasma (PPP) from an anticoagulated blood sample from a subject,
b) processing the PPP to obtain a first composition comprising a fibrinogenic concentrate,
c) obtaining a platelet-rich plasma (PRP) from an anticoagulated blood sample from a subject,
d) activating the platelets in the PRP obtained in c),
e) isolating the serum obtained after platelet activation to obtain a second platelet-free composition comprising thrombin and growth factors,
f) mixing the first composition and the second composition.

2. The method according to claim 1, wherein the mixing of the first composition and the second composition of step f) is performed at a temperature of between 30°C and 44°C.

3. The method according to any of claims 1 or 2, wherein the anticoagulated blood sample of step a) and the anticoagulated blood sample of step c) come from the same subject, and preferably from the same blood draw from that subject.

4. The method according to claim 3, wherein the first composition is obtained from a volume of between 60% and 80% of the draw, and the second composition is obtained from a volume of between 20% and 40% of the draw.

5. The method according to any of claims 1 to 4, wherein the platelet-poor plasma contains a platelet concentration of less than 1 x 10⁵ platelets/µL.

6. The method according to any of claims 1 to 5, wherein the platelet-poor plasma is obtained by means of centrifugation of the anticoagulated blood sample at a speed of at least 1500 g for at least 15 minutes.

7. The method according to any of claims 1 to 6, wherein the first composition comprising the fibrinogenic concentrate is isolated from the platelet-poor plasma by means of a method comprising the following steps:
a) precipitating the proteins in the platelet-poor plasma;
b) optionally cooling during the precipitation of step a);
c) isolating the precipitate obtained in a) or b); and
d) dissolving said precipitate.

8. The method according to claim 7, comprising the following steps:
a) adding an alcohol solution to the plasma in a volume of between 5 and 15% with respect to the volume of said plasma;
b) cooling the mixture obtained in a) at a temperature of between 2°C and 8°C;
c) isolating the precipitate obtained in b) by means of centrifugation at a speed of at least 580 g for at least 5 minutes;
d) dissolving the precipitate obtained in c) by heating.

9. The method according to any of claims 1 to 8, wherein the PRP from which the plasma solution of thrombin and growth factors is obtained, is obtained by means of centrifugation of the anticoagulated blood sample at a speed of between 580 g and 1200 g for at least 8 minutes.

10. The method according to any of claims 1 to 9, wherein platelet activation is carried out by means of the addition of a compound selected from calcium chloride, thrombin and collagen.

11. The method according to claim 10, wherein platelet activation is performed by means of the addition of calcium chloride.

12. The method according to any of claims 1 to 11, wherein the platelet concentration of the first composition is less than 3.5 x 10⁵ platelets/µL.

13. The method according to any of claims 1 to 12, wherein the fibrinogen concentration in the first composition is from 6 to 9 times greater than the fibrinogen concentration in the PPP.

14. The method according to any of claims 1 to 12, wherein the first composition and the second composition are mixed in a volumetric ratio of between 5:1 and 1:5.

15. A fibrin gel obtained by means of the method according to any of claims 1 to 14.

16. The fibrin gel according to claim 15, wherein said gel has a surface adhesion strength of between 200 mN/cm² and 600 mN/cm².

17. The fibrin gel according to any of claims 15 or 16, wherein said gel has a Young's modulus between 11 and 16 KPA, an energy absorption capacity of between 4 and 4.5 mJ/m, and a swelling of between 1.1 and 1.63 measured as the hydrated to dehydrated gel weight ratio.

18. The fibrin gel according to any of claims 15 to 17, wherein the percentage of coagulation is between 67% and 87% measured as the weight of the clot formed after the mixing of additional step f) over the weight of the initial mixture of the first composition and the second composition.

19. The fibrin gel according to any of claims 15 to 18, wherein the platelet concentration is less than 1.075 x 10⁵ platelets/µL.

20. The fibrin gel according to any of claims 15 to 19 for use in medicine.

21. The fibrin gel according to any of claims 15 to 19 for use in the treatment of osteochondral injuries, meniscal injuries or ulcers, particularly vascular ulcers.

22. A fibrin gel for use according to any of claims 20 or 21, wherein the gel is administered to the same patient from whom the blood samples, from which said fibrin gel was obtained, come from.

23. A combination of:
- a first composition, obtained by means of the following steps:
a) obtaining a platelet-poor plasma (PPP) from an anticoagulated blood sample from a subject,
b) processing the PPP to obtain the first composition, comprising a fibrinogenic concentrate;
- and a second composition, obtained by means of the following steps:
c) obtaining a platelet-rich plasma (PRP) from an anticoagulated blood sample from a subject,
d) activating the platelets in the PRP obtained in c),
e) isolating the serum obtained after platelet activation to obtain the second platelet-free composition comprising thrombin and growth factors;
for use in medicine, wherein the use comprises mixing the first composition and the second composition in the patient's body.

24. The combination for use according to claim 23, wherein the mixing of the first composition and the second composition is performed at a temperature of between 30°C and 44°C.
